# EUROPÄISCHE PATENTANMELDUNG

(11) **EP 3 706 052 A1**
(43) Veröffentlichungstag der Anmeldung: **09.09.2020**
(21) Anmeldenummer: 19161707.5
(22) Anmeldetag: 08.03.2019
(51) Int. Cl.: G06Q 10/06, G16H 70/00, G06F 16/23, G06Q 50/22

(54) **KONSISTENTE DATENHALTUNG FÜR EIN PHARMAZEUTISCHES PRODUKT**

(71) Anmelder: Siemens Aktiengesellschaft, 80333 München (DE)
(72) Erfinder: Klocker, Hartmut, 76137 Karlsruhe (DE)

(57) **Zusammenfassung**

Ein pharmazeutisches Produkt (P) wird zunächst entwickelt, sodann erprobt, im Rahmen der Erprobung nachgefertigt und schließlich in Serie gefertigt. Im Rahmen der Entwicklung, der Erprobung, der Nachfertigung und der Serienfertigung des pharmazeutischen Produkts (P) wird auf Daten (D) zurückgegriffen, die in einer für die Entwicklung, die Erprobung, die Nachfertigung und die Serienfertigung des pharmazeutischen Produkts (P) einheitlichen Datenbank (5) gespeichert sind. Auch werden im Rahmen der Entwicklung, der Erprobung, der Nachfertigung und der Serienfertigung des pharmazeutischen Produkts (P) anfallende neue Daten (D) in der einheitlichen Datenbank (5) gespeichert. Im Rahmen der Entwicklung, der Erprobung, der Nachfertigung und der Serienfertigung des pharmazeutischen Produkts (P) wird mittels eines jeweiligen Bussystems (3, 8, 12, 16) mit der einheitlichen Datenbank (5) kommuniziert.

## Beschreibung

Die vorliegende Erfindung geht aus von einem Verfahren zur Datenhaltung für ein pharmazeutisches Produkt, das zunächst entwickelt, sodann erprobt, im Rahmen der Erprobung nachgefertigt und schließlich in Serie gefertigt wird.

Im "Lebenszyklus" eines pharmazeutischen Produkts (= Medikaments) werden von der Entwicklung bis zur Serienproduktion zahlreiche Daten erhoben, die oftmals über alle Phasen des Lebenszyklus des pharmazeutischen Produkts von Bedeutung sind. Beispiele derartiger Daten sind Rezepte und Herstellungsprozeduren, kritische Prozess- und Qualitätsparameter, zu verwendende Materialien, die Spezifikation der Gerätschaften, anlagenspezifische Kenngrößen und andere mehr.

Im Stand der Technik kommt es immer wieder zu Brüchen zwischen den verschiedenen Phasen. Dies führt zu einem Informationsverlust und einem mangelhaften Wissenstransfer.

Die Aufgabe der vorliegenden Erfindung besteht darin, auf einfache Weise eine konsistente Datenhaltung für ein pharmazeutisches Produkt von der Entwicklung bis zur Serienfertigung zu ermöglichen.

Die Aufgabe wird durch ein Verfahren mit den Merkmalen des Anspruchs 1 gelöst. Vorteilhafte Ausgestaltungen des Verfahrens sind Gegenstand der abhängigen Ansprüche 2 bis 6.

Erfindungsgemäß wird ein Verfahren der eingangs genannten Art dadurch ausgestaltet,
- dass im Rahmen der Entwicklung, der Erprobung, der Nachfertigung und der Serienfertigung des pharmazeutischen Produkts auf in einer für die Entwicklung, die Erprobung, die Nachfertigung und die Serienfertigung des pharmazeutischen Produkts einheitlichen Datenbank gespeicherte Daten zurückgegriffen wird und im Rahmen der Entwicklung, der Erprobung, der Nachfertigung und der Serienfertigung des pharmazeutischen Produkts anfallende neue Daten in der einheitlichen Datenbank gespeichert werden und
- dass im Rahmen der Entwicklung, der Erprobung, der Nachfertigung und der Serienfertigung des pharmazeutischen Produkts mittels eines jeweiligen Bussystems mit der einheitlichen Datenbank kommuniziert wird.

Es ist durchaus möglich, dass die Bussysteme für die Entwicklung, die Erprobung, die Nachfertigung und die Serienfertigung des pharmazeutischen Produkts individuell für die Entwicklung, die Erprobung, die Nachfertigung und die Serienfertigung des pharmazeutischen Produkts ausgestaltet sind. In diesem Fall muss lediglich gewährleistet sein, dass die Bussysteme über eine jeweilige Schnittstelle auf die einheitliche Datenbank zurückgreifen und Daten in der einheitlichen Datenbank speichern.

Vorzugsweise ist in der einheitlichen Datenbank festgelegt, welche Daten im Rahmen der Entwicklung, der Erprobung, der Nachfertigung und der Serienfertigung des pharmazeutischen Produkts geändert werden dürfen und welche Daten im Rahmen der Entwicklung, der Erprobung, der Nachfertigung und der Serienfertigung des pharmazeutischen Produkts unverändert beibehalten werden müssen. Dadurch können Fallgestaltungen, bei denen hierzu nicht berechtigte Institutionen - beispielsweise aufgrund einer Fehleingabe eines Menschen - relevante Daten ändern, weitestgehend vermieden werden.

Die in der einheitlichen Datenbank gespeicherten Daten umfassen vorzugsweise Rezepte, Prozeduren und Werkzeuge oder Geräte zur Herstellung des pharmazeutischen Produkts, Prozessparameter, Zielgrößen, Gebrauchsmaterialien und Verbrauchsmaterialien. Vorzugsweise sind die in der einheitlichen Datenbank gespeicherten Daten hierbei in der einheitlichen Datenbank anlagenneutral, skalierbar und/oder generisch hinterlegt. Dadurch ist auf einfache Weise ein Transfer beispielsweise einer Fertigung von einer Anlage zu einer anderen Anlage möglich.

Vorzugsweise bleiben bei einer Änderung von Daten die vorherigen Daten in der einheitlichen Datenbank im Sinne einer Historie gespeichert, so dass auf sie zugreifbar ist. Dadurch ist es möglich, beispielsweise zu Dokumentationszwecken auf diese Daten zuzugreifen oder auch, falls sich eine Änderung als fehlerhaft erwiesen hat, die geänderten Daten zu löschen oder als fehlerhaft zu markieren, so dass die vorherigen Daten wieder gültig werden.

Vorzugsweise ist vorgesehen, dass bei einem normalen Abruf nur auf die in der einheitlichen Datenbank gespeicherten aktuellen Daten zugegriffen wird und nur bei einem speziellen Abruf auf die im Sinne einer Historie in der einheitlichen Datenbank gespeicherten Daten zugegriffen wird. Dadurch wird der Zugriff auf die in der einheitlichen Datenbank gespeicherten Daten vereinfacht.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: ein Übersichtsbild,
- FIG 2: eine Datenstruktur einer Datenbank und
- FIG 3: verkettete Datensätze.

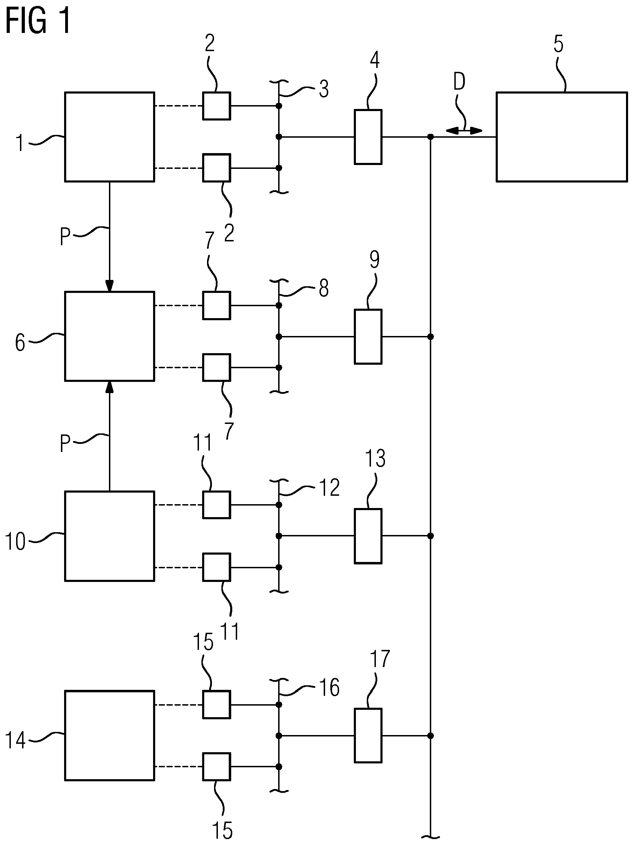

Entsprechend der Darstellung in FIG 1 wird ein pharmazeutisches Produkt P - insbesondere ein Arzneimittel - zunächst in einem Entwicklungslabor 1 entwickelt. In dem Entwicklungslabor 1 werden zur Entwicklung des Produkts P im Rahmen vieler Versuche viele verschiedene Substanzen hergestellt, bis eine - zumindest vorläufige - Zusammensetzung des Produkts P feststeht. Die Substanzen werden jeweils in einem kleinen, labortechnischen Maßstab hergestellt. Im Rahmen der Entwicklung des Produkts P werden unter Verwendung mindestens eines Rechners 2 - in der Regel mehrerer Rechner 2 - in erheblichem Umfang Daten D erstellt und verwertet. Die Daten D werden teilweise zur Verfügung gestellt und fallen teilweise im Rahmen der Entwicklung des Produkts P an.

Soweit die Daten D zur Verfügung stehen, werden sie von dem Rechner 2 bzw. den Rechnern 2 aus über ein Bussystem 3 und eine Schnittstelle 4 aus einer Datenbank 5 abgerufen. Soweit die Daten D erstellt werden oder geändert werden, werden sie von dem Rechner 2 bzw. den Rechnern 2 aus über das Bussystem 3 und die Schnittstelle 4 in der Datenbank 5 gespeichert. Der Rechner 2 bzw. die Rechner 2 kommuniziert/kommunizieren somit mittels des Bussystems 3 und die Schnittstelle 4 mit der Datenbank 5. Das Bussystem 3 kann individuell für die Entwicklung des pharmazeutischen Produkts P, im Ergebnis also für die Kommunikation des Rechners 2/der Rechner 2 mit der Datenbank 5, ausgestaltet sein.

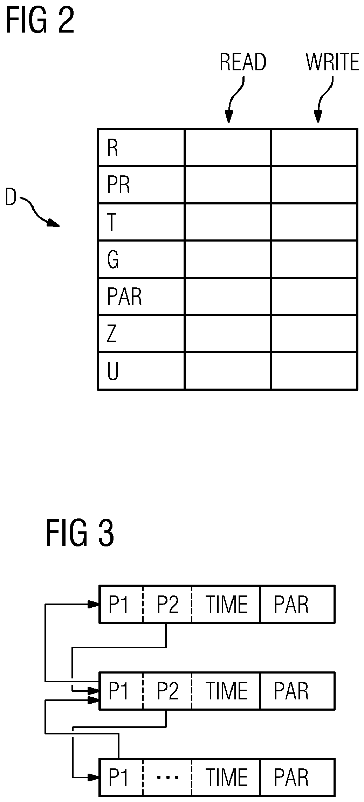

Die zur Verfügung gestellten Daten können entsprechend der Darstellung in FIG 2 z.B. eine anfängliche Rezeptur R und eine anfängliche Prozedur PR zur Herstellung des Produkts P umfassen. Sowohl die Rezeptur R als auch die Prozedur PR können im Rahmen der Entwicklung geändert werden. Die zur Verfügung gestellten Daten können weiterhin beispielsweise Vorschriften umfassen, welche Werkzeuge T oder Geräte G oder allgemein Gebrauchsmaterialien zur Herstellung des pharmazeutischen Produkts P verwendet werden sollen und mit welchen Prozessparametern PAR die Werkzeuge T oder Geräte G betrieben werden sollen. Auch können Zielgrößen Z vorgegeben werden, welche das hergestellte Produkt P erfüllen und einhalten soll. Auch kann vorgegeben werden oder sein, welche Verbrauchsmaterialien U zur Herstellung des Produkts P verwendet werden sollen. Die genannten Daten können, soweit möglich und erforderlich, in der Datenbank 5 anlagenneutral, skalierbar und/oder generisch hinterlegt sein.

Nach der erstmaligen Entwicklung des Produkts P erfolgt in einer Klinik 6 eine klinische Studie. Gegebenenfalls können auch mehrere derartige Studien erfolgen. Im Rahmen der Studie werden unter Verwendung mindestens eines Rechners 7 - in der Regel mehrerer Rechner 7 - ebenfalls in erheblichem Umfang Daten D erstellt und verwertet. Wie zuvor werden die Daten D, soweit sie zur Verfügung stehen, von dem Rechner 7 bzw. den Rechnern 7 aus über ein Bussystem 8 und eine Schnittstelle 9 aus der Datenbank 5 abgerufen. Soweit die Daten D erstellt werden oder geändert werden, werden sie von dem Rechner 3 bzw. den Rechnern 3 aus über das Bussystem 8 und die Schnittstelle 9 in der Datenbank 5 gespeichert. Der Rechner 7 bzw. die Rechner 7 kommuniziert/kommunizieren somit mittels des Bussystems 8 und die Schnittstelle 9 mit der Datenbank 5. Das Bussystem 8 kann individuell für die Erprobung des pharmazeutischen Produkts P, im Ergebnis also für die Kommunikation des Rechners 7/der Rechner 7 mit der Datenbank 5, ausgestaltet sein.

Im Rahmen der klinischen Studie werden in der Regel zusätzliche Mengen des Produkts P benötigt. In einer kleinen Herstellungsanlage 10 werden daher die entsprechenden Mengen des Produkts P hergestellt. Die kleine Herstellungsanlage 10 weist eine Größe auf, die einerseits größer als das Entwicklungslabor 1 ist, so dass die in der kleinen Herstellungsanlage 10 in gleicher Zeit herstellbare Menge des Produkts P weit oberhalb derjenigen Menge liegt, die in dem Entwicklungslabor 1 herstellbar ist. Es erfolgt also eine Herstellung mit einer Menge oberhalb eines kleinen, labortechnischen Maßstabes. Dieser Vorgang wird im Rahmen der vorliegenden Erfindung als Nachfertigung bezeichnet.

Im Rahmen der Herstellung des Produkts P in der kleinen Herstellungsanlage 10 werden unter Verwendung mindestens eines Rechners 11 - in der Regel mehrerer Rechner 11 - in erheblichem Umfang Daten D erstellt und verwertet. Die Daten D werden teilweise zur Verfügung gestellt und fallen teilweise im Rahmen der Herstellung des Produkts P an. Soweit die Daten D zur Verfügung stehen, werden sie von dem Rechner 11 bzw. den Rechnern 11 aus über ein Bussystem 12 und eine Schnittstelle 13 aus der Datenbank 5 abgerufen. Soweit die Daten D erstellt werden oder geändert werden, werden sie von dem Rechner 11 bzw. den Rechnern 11 aus über das Bussystem 12 und die Schnittstelle 13 in der Datenbank 5 gespeichert. Der Rechner 11 bzw. die Rechner 11 kommuniziert/kommunizieren somit mittels des Bussystems 12 und die Schnittstelle 13 mit der Datenbank 5. Das Bussystem 12 kann individuell für die Fertigung des pharmazeutischen Produkts P, im Ergebnis also für die Kommunikation des Rechners 11/der Rechner 11 mit der Datenbank 5, ausgestaltet sein.

Nach der endgültigen Erprobung des Produkts P erfolgt - sofern die Erprobung erfolgreich war - nach einem oftmals langwierigen Genehmigungsverfahren eine Freigabe des Produkts P. Damit ist die Voraussetzung geschaffen, dass das Produkt P in einer großen Herstellungsanlage 14 in industriellem Maßstab hergestellt wird. Die große Herstellungsanlage 14 weist eine Größe auf, die größer als die kleine Herstellungsanlage 10 ist, so dass die in der großen Herstellungsanlage 14 in gleicher Zeit herstellbare Menge des Produkts P weit oberhalb derjenigen Menge liegt, die in der kleinen Herstellungsanlage 10 herstellbar ist. Es erfolgt also eine industrielle Herstellung (Serienfertigung) des Produkts P.

Im Rahmen der Herstellung des Produkts P in der großen Herstellungsanlage 14 werden unter Verwendung mindestens eines Rechners 15 - in der Regel mehrerer Rechner 15 - in erheblichem Umfang Daten D erstellt und verwertet. Die Daten D werden teilweise zur Verfügung gestellt und fallen teilweise im Rahmen der Herstellung des Produkts P an. Soweit die Daten D zur Verfügung stehen, werden sie von dem Rechner 15 bzw. den Rechnern 15 aus über ein Bussystem 16 und eine Schnittstelle 17 aus der Datenbank 5 abgerufen. Soweit die Daten D erstellt werden oder geändert werden, werden sie von dem Rechner 15 bzw. den Rechnern 15 aus über das Bussystem 16 und die Schnittstelle 17 in der Datenbank 5 gespeichert. Der Rechner 15 bzw. die Rechner 15 kommuniziert/kommunizieren somit mittels des Bussystems 16 und die Schnittstelle 17 mit der Datenbank 5. Das Bussystem 16 kann individuell für die Serienfertigung des pharmazeutischen Produkts P, im Ergebnis also für die Kommunikation des Rechners 15/der Rechner 15 mit der Datenbank 5, ausgestaltet sein.

Die Datenbank 5 ist eine für die Entwicklung, die Erprobung, die Nachfertigung und die Serienfertigung des pharmazeutischen Produkts P einheitliche Datenbank. Auf ihre Daten D wird im Rahmen der Entwicklung, der Erprobung, der Nachfertigung und der Serienfertigung des pharmazeutischen Produkts P zurückgegriffen. Dies gilt sowohl für bereits in der Datenbank 5 gespeicherte Daten als auch für neu anfallende Daten. Diese werden in diesem Fall in der Datenbank 5 gespeichert. Die Daten D stehen somit einheitlich für die Entwicklung, die Erprobung, die Nachfertigung und die Serienfertigung des pharmazeutischen Produkts P und die hierbei verwendeten Rechner 2, 7, 11, 15 zur Verfügung.

Es ist denkbar, dass die Daten D in der Datenbank 5 derart hinterlegt sind, dass von jedem der Rechner 2, 7, 11, 15 aus auf alle Daten D zugegriffen werden kann und dass jeder Zugriff sowohl lesend als auch schreibend sein kann. In der Regel ist entsprechend der Darstellung in FIG 2 jedoch registriert, mittels welcher Rechner 2, 7, 11, 15 lesend ("READ") und mittels welcher Rechner 2, 7, 11, 15 schreibend ("WRITE") auf welche Daten D zugegriffen werden kann. Es ist also festgelegt, welche Daten D im Rahmen der Entwicklung, der Erprobung, der Nachfertigung und der Serienfertigung des pharmazeutischen Produkts P jeweils geändert werden dürfen und welche Daten D im Rahmen der Entwicklung, der Erprobung, der Nachfertigung und der Serienfertigung des pharmazeutischen Produkts P jeweils unverändert beibehalten werden müssen. Gegebenenfalls können die entsprechenden Rechte einzeln für die einzelnen Rechner 2, 7, 11, 15 des Entwicklungslabors 1, der Klinik 6 sowie der kleinen und der großen Herstellungsanlage 10, 14 vergeben sein.

Es ist weiterhin möglich, dass in der Datenbank 5 jeweils nur die zuletzt gespeicherten Daten D gespeichert werden, dass also, wenn ein bestimmter Wert geändert wird, nur der geänderte Wert weiterhin gespeichert wird, während der vorher gültige Wert verdrängt wird. Vorzugsweise bleiben bei einer Änderung von Daten D jedoch die vorherigen Daten in der Datenbank 5 im Sinne einer Historie gespeichert, so dass auch zu einem späteren Zeitpunkt noch auf sie zugreifbar ist. Beispielsweise kann, wie in FIG 3 exemplarisch für einen der Prozessparameter PAR angedeutet, das jeweilige Datum D in der Datenbank 5 zusammen mit einem Header 18 abgespeichert sein, wobei der jeweilige Header 18 zum einen einen Zeitstempel TIME und zum anderen einen Verweis P1 (Pointer P1) auf den jeweils vorherigen Wert des entsprechenden Datums D enthält. Ältere Daten wiederum können in ihrem Header 18 zusätzlich zu dem Zeitstempel TIME und dem Verweis P1 auf den jeweils vorherigen Wert des entsprechenden Datums D weiterhin einen Verweis P2 (Pointer P2) auf den jeweils nachfolgenden Wert des entsprechenden Datums D enthalten.

Bei einem normalen Abruf der Daten D wird in diesem Fall nur auf die aktuellen Daten D zugegriffen, also auf diejenigen Daten D mit dem jüngsten Datum. Bei einem speziellen Abruf - beispielsweise bei einer Ergänzung des Abrufs durch den Code "HISTORIE" - wird hingegen nicht nur der aktuelle Wert des jeweiligen Datums D abgerufen, sondern auch dessen Historie.

Zusammengefasst betrifft die vorliegende Erfindung somit folgenden Sachverhalt:
Ein pharmazeutisches Produkt P wird zunächst entwickelt, sodann erprobt, im Rahmen der Erprobung nachgefertigt und schließlich in Serie gefertigt. Im Rahmen der Entwicklung, der Erprobung, der Nachfertigung und der Serienfertigung des pharmazeutischen Produkts P wird auf Daten D zurückgegriffen, die in einer für die Entwicklung, die Erprobung, die Nachfertigung und die Serienfertigung des pharmazeutischen Produkts P einheitlichen Datenbank 5 gespeichert sind. Auch werden im Rahmen der Entwicklung, der Erprobung, der Nachfertigung und der Serienfertigung des pharmazeutischen Produkts P anfallende neue Daten D in der einheitlichen Datenbank 5 gespeichert. Im Rahmen der Entwicklung, der Erprobung, der Nachfertigung und der Serienfertigung des pharmazeutischen Produkts P wird mittels eines jeweiligen Bussystems 3, 8, 12, 16 mit der einheitlichen Datenbank 5 kommuniziert.

Die vorliegende Erfindung weist viele Vorteile auf. Insbesondere kann auf einfache und zuverlässige Weise eine konsistente Datenhaltung über den gesamten "Lebenszyklus" des pharmazeutischen Produkts P erreicht werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Entwicklungslabor
- 2, 7, 11, 15: Rechner
- 3, 8, 12, 16: Bussysteme
- 4, 9, 13, 17: Schnittstellen
- 5: Datenbank
- 6: Klinik
- 10: kleine Herstellungsanlage
- 14: große Herstellungsanlage
- 18: Header

- D: Daten
- G: Geräte
- P: Produkt
- P1, P2: Verweise
- PAR: Parameter
- PR: Prozedur
- R: Rezeptur
- T: Werkzeuge
- TIME: Zeitstempel
- U: Verbrauchsmaterialien
- Z: Zielgrößen

## Patentansprüche

1. Verfahren zur Datenhaltung für ein pharmazeutisches Produkt (P), das zunächst entwickelt, sodann erprobt, im Rahmen der Erprobung nachgefertigt und schließlich in Serie gefertigt wird,
- wobei im Rahmen der Entwicklung, der Erprobung, der Nachfertigung und der Serienfertigung des pharmazeutischen Produkts (P) auf in einer für die Entwicklung, die Erprobung, die Nachfertigung und die Serienfertigung des pharmazeutischen Produkts (P) einheitlichen Datenbank (5) gespeicherte Daten (D) zurückgegriffen wird und im Rahmen der Entwicklung, der Erprobung, der Nachfertigung und der Serienfertigung des pharmazeutischen Produkts (P) anfallende neue Daten (D) in der einheitlichen Datenbank (5) gespeichert werden und
- wobei im Rahmen der Entwicklung, der Erprobung, der Nachfertigung und der Serienfertigung des pharmazeutischen Produkts (P) mittels eines jeweiligen Bussystems (3, 8, 12, 16) mit der einheitlichen Datenbank (5) kommuniziert wird.

2. Verfahren nach Anspruch 1,
**dadurch gekennzeichnet,**
**dass** die Bussysteme (3, 8, 12, 16) für die Entwicklung, die Erprobung, die Nachfertigung und die Serienfertigung des pharmazeutischen Produkts (P) individuell für die Entwicklung, die Erprobung, die Nachfertigung und die Serienfertigung des pharmazeutischen Produkts (P) ausgestaltet sind und über eine jeweilige Schnittstelle (4, 9, 13, 17) auf die einheitliche Datenbank (5) zurückgreifen und Daten (D) in der einheitlichen Datenbank (5) speichern.

3. Verfahren nach Anspruch 1 oder 2,
**dadurch gekennzeichnet,**
**dass** in der einheitlichen Datenbank (5) festgelegt ist, welche Daten (D) im Rahmen der Entwicklung, der Erprobung, der Nachfertigung und der Serienfertigung des pharmazeutischen Produkts (P) geändert werden dürfen und welche Daten (D) im Rahmen der Entwicklung, der Erprobung, der Nachfertigung und der Serienfertigung des pharmazeutischen Produkts (P) unverändert beibehalten werden müssen.

4. Verfahren nach Anspruch 1, 2 oder 3,
**dadurch gekennzeichnet,**
**dass** die in der einheitlichen Datenbank (5) gespeicherten Daten (D) Rezepte (R), Prozeduren (PR) und Werkzeuge (T) oder Geräte (G) zur Herstellung des pharmazeutischen Produkts (P), Prozessparameter (PAR), Zielgrößen (Z), Gebrauchsmaterialien und Verbrauchsmaterialien (U) umfassen.

5. Verfahren nach einem der obigen Ansprüche,
**dadurch gekennzeichnet,**
**dass** bei einer Änderung von Daten (D) die vorherigen Daten (D) in der einheitlichen Datenbank (5) im Sinne einer Historie gespeichert bleiben, so dass auf sie zugreifbar ist.

6. Verfahren nach Anspruch 5,
**dadurch gekennzeichnet,**
**dass** bei einem normalen Abruf nur auf die in der einheitlichen Datenbank (5) gespeicherten aktuellen Daten (D) zugegriffen wird und nur bei einem speziellen Abruf auf die im Sinne einer Historie in der einheitlichen Datenbank (5) gespeicherten Daten (D) zugegriffen wird.
